Europäisches Patentamt

European Patent Office

Office européen des brevets

(19)

(11) Numéro de publication: **0 128 848**
**B1**

(12)

# FASCICULE DE BREVET EUROPEEN

(45) Date de publication du fascicule du brevet:
**07.10.87**

(21) Numéro de dépôt: **84450013.2**

(22) Date de dépôt: **18.05.84**

(51) Int. Cl.⁴: **H 04 R 25/00,** H 04 R 29/00,
A 61 B 5/12

(54) **Banc d'essai pour chaîne électro-acoustique et notamment pour appareil de correction auditive.**

(30) Priorité: **27.05.83 FR 8309120**

(43) Date de publication de la demande:
**19.12.84 Bulletin 84/51**

(45) Mention de la délivrance du brevet:
**07.10.87 Bulletin 87/41**

(84) Etats contractants désignés:
**CH DE IT LI**

(56) Documents cités:
**EP - A - 0 010 169**
**US - A - 3 922 506**
**US - A - 4 095 057**
**US - A - 4 099 035**

**JOURNAL OF THE BRITISH INSTITUTION OF RADIO
ENGINEERS, vol. 11, no. 5, mai 1951, Londres (GB); G.L.
HAMBURGER:"The production model of the automatic
A.F. response curve tracer", pages 165-201**

(73) Titulaire: **Michas, Frédéric, 86 Avenue Trespoey,
F-64000 Pau (FR)**

(72) Inventeur: **Michas, Frédéric, 86 Avenue Trespoey,
F-64000 Pau (FR)**

(74) Mandataire: **Ravina, Bernard, Cabinet Bernard
RAVINA 24, boulevard Riquet, F-31000 Toulouse (FR)**

## Description

La présente invention a pour objet un banc d'essai pour chaînes électro-acoustiques et notamment pour appareil de correction auditive à l'usage de personnes malentendantes.

Pour rétablir une audition normale chez les personnes malentendantes à l'aide d'appareil de correction auditive il est nécessaire dans un premier temps de corriger les distorsions qu'apporte ce genre d'appareil à la reproduction des sons du spectre audible et dans un second temps d'adapter cet appareil à l'ouie du patient.

Dans un premier temps ce genre d'appareil doit donc être réglé en sorte d'amplifier chaque fréquence du spectre sonore de manière identique et donc doit être réglé de manière à ne pas reproduire certaines de ces fréquences avec un surcroît d'intensité. De plus ce genre d'appareil doit reproduire assez fidèlement les bruits secs et de ce fait doit présenter une bonne réponse aux signaux impulsionnels.

Il est à noter également que l'analyse de la réponse de ce genre d'appareil à des signaux carrés de fréquence déterminée renseigne utilement l'homme de l'art sur le fonctionnement de la chaîne et notamment sur la réponse aux transitoires ainsi que sur les pics de résonnance. A ce stade pour faciliter le réglage de l'appareil il est souhaitable que toutes les modifications, apportées à celui-ci en vue d'en atténuer les défauts, soient immédiatement perceptibles à l'homme de l'art. De plus il est necessaire que l'homme de l'art puisse comparer à chaque instant la courbe de réponse de l'appareil aux signaux sonores perçus par le dit appareil.

Lorsque l'homme de l'art a procédé à ces différents réglages il peut adapter l'appareil de correction auditive à l'ouie du patient. Dans un premier temps l'homme de l'art, avec l'aide du patient, détermine les zones du spectre sonore pour lesquelles une atténuation est à apporter et les zones du spectre sonore qui doivent subir une amplification, le but étant de restituer une audition la plus normale possible.

En outre à ce stade de l'adaptation de l'appareil il est souhaitable de confronter le patient aux sons de l'environnement naturel en sorte d'affiner le réglage. En conclusion on comprend que la restitution d'une audition normale chez une personne malentendante dépend en grande partie de la qualité du réglage effectué sur l'appareil de correction auditive.

On connait déjà du document EP-A-010 169 un dispositif de mesure, de contrôle et de réglage de la pression acoustique maximale d'un appareil de correction auditive. Un tel dispositif comprend une source sonore couplée phoniquement au microphone de l'appareil et un dispositif électro-acoustique de contrôle et de mesure couplé phoniquement au transducteur de l'appareil à corriger.

Le dispositif de contrôle est constitué en combinaison par un microphone qui perçoit les sons émis par le transducteur de l'élément d'adaptation à l'oreille, par un amplificateur associé électriquement au microphone et par un appareil indicateur du niveau sonore.

Cet appareil est également équipé d'un oscilloscope pour la visualisation du signal électrique représentatif du son perçu par le microphone. Cependant, avec un tel dispositif, on ne peut procéder facilement à l'atténuation des distorsions de l'appareil de correction et il ne peut être procédé à l'adaptation du dit appareil à l'ouie du malentendant, en raison dufait que ce dispositif n'est pas équipé de moyens pour modéliser l'appareil de correction du malentendant et en raison du fait qu'il n'est pas équipé de moyens pour visualiser simultanément le signal reçu par l'appareil et le signal délivré par l'appareil afin d'établir une comparaison entre ces deux signaux.

La présente invention a donc pour objet un banc d'essai pour chaînes électro-acoustiques et notamment pour appareil de correction auditive permettant de faciliter les réglages de ce gernre d'appareil.

A cet effet, le banc d'essai selon la présente invention pour la mise au point des chaînes électro-acoustiques et notamment des appareils de correction auditive, en sorte que ceux-ci restituent avec fidélité le timbre et la hauteur des sons perçus, et restituent ces sons avec plus ou moins d'intensité en fonction de l'audition du malentendant, comporte:

– plusieurs sources de production d'un signal électrique,

– au moins un moyen de transformation de ce signal électrique en signal sonore, le dit signal sonore étant reçu par l'appareil,

– au moins un moyen pour transformer en signal électrique le signal sonore sortant de l'appareil,

– et un moyen de visualisation des signaux électriques, et est caractérisé par:

– au moins un ensemble de moyens pour modéliser analogiquement un appareil de correction auditive propre au malentendant,

– par au moins un moyen pour transformer en signal électrique le signal sonore entrant dans l'appareil,

– par l'utilisation du moyen (7) de visualisation pour visualiser les signaux électriques délivrés par l'ensemble de moyens (4) pour modéliser l'appareil de correction auditive propre au malentendant, par le moyen (5) pour transformer en signal électrique le signal sonore recu par l'appareil (1), et par le moyen (6) pour transformer en signal électrique le signal sonore émis par l'appareil (1),

– et par des moyens de mémorisation des signaux électriques visualisés.

Selon une autre caractéristique de l'invention, les signaux sont visualisés suivant une échelle linéaire en sorte que les défauts ou distorsions, apportés par cet appareil à la reproduction des sons, soient appréciés à leur juste valeur.

D'autres avantages et caractéristiques de l'invention apparaîtront à la lecture de la description d'une forme préférentielle de réalisation donnée à titre d'exemple non limitatif en se référant aux dessins annexés en lesquels:

— La figure 1 est un schéma synoptique du banc d'essai selon l'invention,

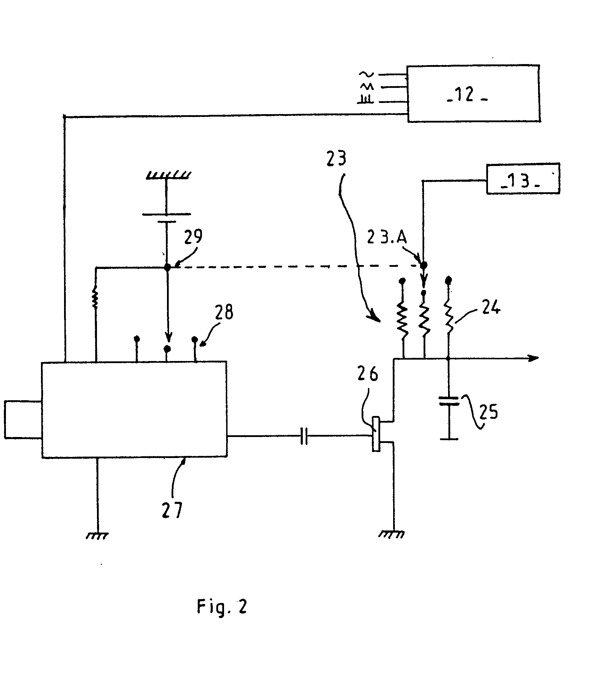

— la figure 2 est un schéma électronique de la base de temps du moyen de visualisation du banc d'essai,

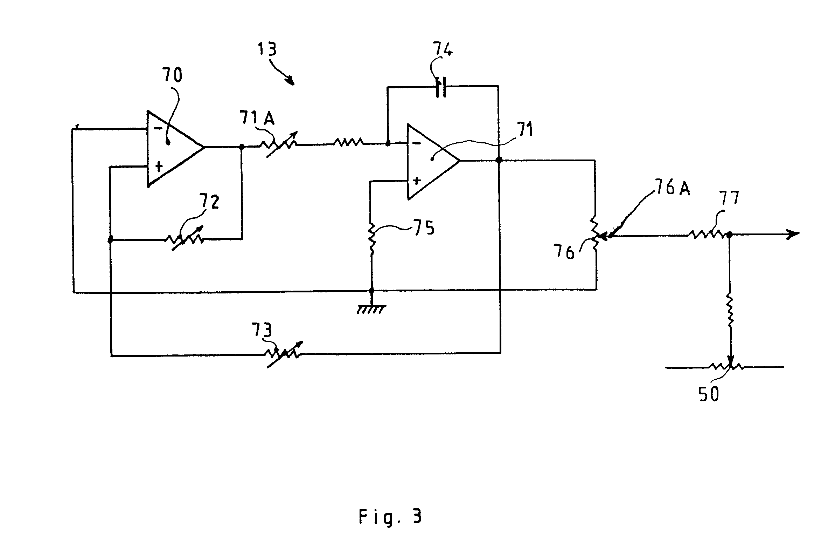

— la figure 3 est un schéma électronique du générateur de balayage équipant le banc d'essai,

— la figure 4 est une vue de face du banc d'essai selon l'invention.

— La figure 5 est une vue en perspective d'un coupleur acoustique équipant le banc d'essai selon l'invention.

— La figure 6 est une vue en coupe suivant la ligne AA de la fig. 5.

— La figure 7 est une vue de profil d'une bague équipant le coupleur acoustique selon la fig. 5.

Tel que représenté le banc d'essai selon l'invention pour la mise au point des chaînes électroacoustiques et notamment des appareils 1 de correction auditive en sorte que ceux-ci restituent avec fidélité le timbre et la hauteur des sons perçus et restituent ces sons avec plus ou moins d'intensité en fonction de l'audition des malentendants est constitué:

— par plusieurs sources 2 de production d'un signal électrique,

— par au moins un moyen 3 de transformation du signal électrique en signal sonore, le dit signal sonore étant reçu par l'appareil 1,

— par au moins un ensemble de moyens 4 pour modéliser un appareil de correction auditive propre au malentendant,

— par au moins un moyen 5 pour transformer en signal électrique le signal sonore qui est émis par le moyen 3 et donc reçu par l'appareil 1,

— par au moins un moyen 6 pour transformer en signal électrique le signal sonore émis par l'appareil,

— par au moins un moyen de visualisation 7 des différents signaux électriques,

— et par au moins des moyens de mémorisation de ces différents signaux électriques visualisés.

Ainsi l'homme de l'art pourra apprécier les défauts et distorsions de l'appareil ce qui facilite sa mise au point, et par comparaison de la réponse mémorisée du modèle avec la réponse corrigée de l'appareil sera en mesure d'ajuster le dit appareil à l'audition du patient.

Selon une forme préférentielle de réalisation les sources 2 sont constituées par au moins un moyen générant un signal audio fréquence et par au moins un moyen générant un signal carré, un signal triangulaire un train d'impulsions et un signal sinusoidal de fréquence fixe ou variable.

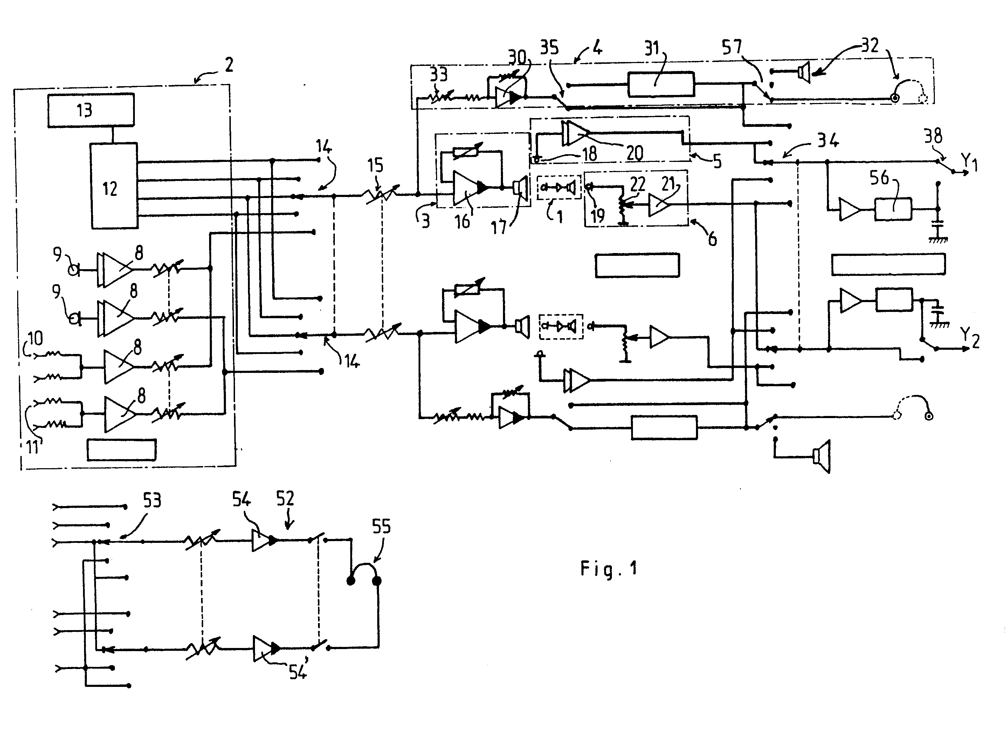

Selon la forme préférentielle de réalisation, le banc d'essai est équipé de plusieurs moyens générant chacun un signal audio fréquence. Comme on peut le voir en fig. 1 chacun de ces moyens est constitué par un étage d'amplification 8 attaqué par un microphone 9 ou par un récepteur radiophonique 10 ou par un lecteur 11 de bande magnétique préenregistrée. De préférence, les signaux carrés, triangulaires, le train d'impulsions et le signal sinusoidal de fréquence fixe ou variable sont délivrés par un générateur de fonctions 12 connu en soi. Un moyen 13 est associé au générateur de fonctions 12 pour commander la fréquence des signaux issus de ce générateur.

Ce moyen 13 est un générateur qui peut délivrer soit une tension continue d'amplitude ajustable ou soit une tension croissante et décroissante cycliquement. Le générateur 12 de production des signaux carrés, triangulaires, du train d'impulsions et du signal sinusoidal comporte quatre sorties affectées chacune à un de ces signaux. Un des signaux délivré par ce générateur ou par les sources audio-fréquence est sélectionné pour attaquer le moyen 3 de production du signal sonore reçu par l'appareil de correction ou pour attaquer le moyen 4.

A cet effet, entre les sources 2 et les moyens 3 et 4 est disposé un sélecteur 14 miltipositions connu en soi. De plus pour régler le volume des signaux dans les moyens 3 et 4 un potentiomètre 15 est disposé entre le sélecteur multipositions 14 et ces moyens 3 et 4. En fonction des besoins, l'homme de l'art sélectionne un des signaux audio fréquence ou les signaux carrés, triangulaires, le train d'impulsions ou le signal sinusoidal.

Les signaux audio fréquence sont par exemple sélectionnés lorsque l'homme de l'art veut créer un modèle d'appareil tandis que les signaux carrés, triangulaires, sinusoidaux et le train d'impulsions sont sélectionnés de préférence lorsqu'il procède à la mise au point de l'appareil pour réduire les défauts ou les distorsions.

La forme visualisée de la réponse de l'appareil au signal carré dont la fréquence est de préférence égale à 200 HZ est représentative du fonctionnement global de celui-ci. Il est à noter également que l'observation de la forme de la réponse permet la détection des pics de résonnance et apporte des précisions sur les réponses de l'appareil aux transitoires. La forme visualisée de la réponse de l'appareil au train d'impulsions est représentative de la reproduction des bruits secs. Par l'observation de cette forme de réponse l'homme de l'art sera en mesure de déterminer si l'appareil restitue ce genre de bruit avec ou non une légère résonnance.

De préférence, le signal sinusoidal est vobulé c'est à dire que la fréquence de ce signal croît et décroît de manière cyclique. Cette variation de fréquence du signal sinusoidal est commandée par le générateur 13 qui produit à cet effet un signal triangulaire injecté dans le générateur 12. Il est à noter que la vobulation du signal est effectuée à amplitude constante et que cette amplitude peut être réglée par l'opérateur. De préférence la fréquence du signal sinusoidal est variable entre 20 HZ et 20 000 HZ.

L'observation de la réponse de l'appareil à ce signal permet de déceler les variations d'amplitude et les surintensités suivant une plage de fréquence étudiée. De plus, l'observation de la symétrie de l'enveloppe de la forme de la réponse permet de déceler un éventuel écretage de l'amplificateur de l'appareil de correction. Il est à noter également que le générateur de fonctions peut délivrer un signal sinusoidal de fréquence fixe.

Dans ce but le moyen 13 délivre une tension continue dont la valeur commande la fréquence su signal issu du générateur 12. Le signal sélectionné attaque le moyen 3, et le moyen 4. Le moyen 3 est constitué par un amplificateur de puissance 16 et par un transducteur électro-acoustique 17 par exemple un haut parleur. L'amplificateur 16 et le haut parleur 17 sont du type de ceux présentant une bonne réponse fréquentielle en sorte de n'apporter aucune distorsion lors de la transformation du signal électrique en signal sonore.

A titre d'exemple nullement limitatif l'amplificateur 16 est un circuit intégré du type «LM324» associé à deux transistors montés en Push pull et le haut parleur est du type de ceux commercialisés par «Sony» sous la référence «MDR3 WALKMAN». Le signal sonore émis par le haut parleur 17 est reçu d'une part par le microphone de l'appareil et d'autre part par le moyen 5. Ce moyen transforme le signal sonore en signal électrique qui de ce fait pourra être visualisé par le moyen 7.

Les moyens 5 et 6 sont constitués chacun par un microphone et par un étage d'amplification attaqué par ce microphone. Le signal électrique est prélevé à la sortie de l'amplificateur et est injecté dans le moyen 7 de visualisation. Le haut parleur 17 est couplé acoustiquement au microphone 18 du moyen 5 et au microphone de l'appareil de correction auditive et le haut parleur de cet appareil est couplé acoustiquement au microphone 19 du moyen 6.

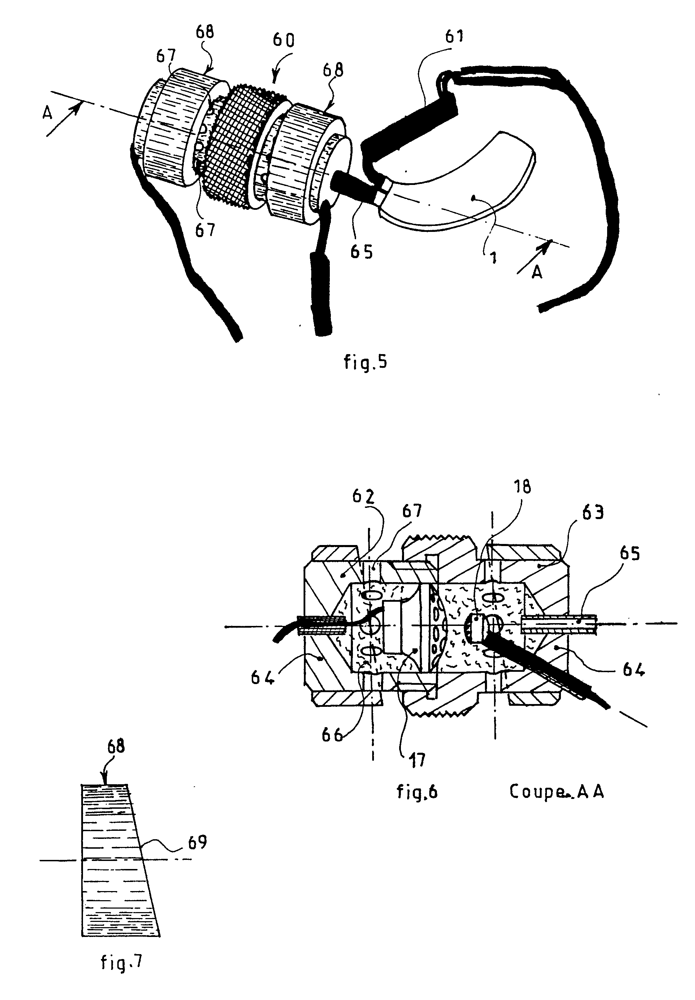

A cet effet, le banc d'essai comporte un premier coupleur acoustique 60 coopérant avec le haut parleur 17, le microphone 18 et le microphone de l'appareil 1 et un second coupleur acoustique 61 coopérant avec le haut parleur du dit appareil 1 et le microphone 19 du moyen 16. Le coupleur 60 comme on peut le voir en fig. 5 et 6 est constitué par deux enceintes tubulaires 62 et 63 comportant chacune un fond 64 et une embouchure filetée pour se visser l'une avec l'autre. Dans l'enceinte 62 est disposé le haut parleur 17 et dans l'enceinte 63 le microphone 18.

Le fond 64 de l'enceinte d'un orifice de passage des cordons électriques du haut parleur 17 et le fond 64 de l'enceinte 63 est percé de deux orifices pour le passage des cordons électriques du microphone 18 et pour le passage d'un tube souple 65 reliant acoustiquement l'enceinte 63 au microphone de l'appareil 1.

Dans les enceintes 62 et 63 est introduit de la mousse 66 ou toute autre matière analogue pour éviter tous phénomènes de resonnance acoustique. Pour régler acoustiquement le coupleur acoustique et notamment le niveau sonore des sons graves chaque enceinte 62 et 63 est dotée d'une série d'orifices transversaux 67 angulairement espacés ménagés dans sa paroi cylindrique et d'une bague 68 emmanchée en frottement doux sur la dite paroi. Cette bague vient obstruer plus ou moins les orifices 67. De préférence le bord 69 de la bague qui vient en regard des orifices est incliné par rapport au plan normal à l'axe de la dite bague. Cette disposition permet d'obstruer certains orifices 67 et d'en dégager d'autres.

Le coupleur acoustique 61 est constitué par un tube souple terminé par deux embouts d'extrémité pour se raccorder d'une part à l'embout de l'appareil 1 et d'autre part au microphone 19 du moyen 6. Les moyens 5 et 6 sont constitués d'éléments connus en soi, présentant une bonne réponse fréquentielle pour éviter toute distorsion dans la transformation du signal sonore en signal électrique.

A titre d'exemple non limitatif, le microphone 18 du moyen 5 et le microphone 19 du moyen 6 sont commercialisés par «LEM» sous la référence «EM 76». L'amplificateur 20 du moyen 5 et l'amplificateur du moyen 6 sont constitués chacun par un circuit intégré «LM 324».

La simplicité de constitution des moyens 5 et 6, ceux-ci n'étant pas pourvus de dispositif de correction du signal électrique, conduit à diminuer les temps de réponse de ces moyens. Comme on peut le voir en fig. 1, le moyen 6 est doté entre le microphone 19 et l'amplificateur 21 d'un potentiomètre 22 pour ajuster le niveau du signal injecté dans cet amplificateur et par conséquent le niveau du signal électrique injecté dans le moyen 7. Les deux signaux électriques présents respectivement à la sortie du moyen 5 et à la sortie du moyen 6 sont représentatifs du signal sonore injecté dans l'appareil et du signal sonore délivré par celui-ci c'est à dire de la réponse de cet appareil. De préférence ces deux signaux sont visualisés simultanément ce qui facilite la comparaison entre ceux-ci.

A cet effet, le moyen 7 de visualisation comporte deux entrées référencées respectivement Y1 et Y2. Ce moyen de visualisation est de préférence constitué par un tube cathodique à double trace associé à un circuit électronique assurant son fonctionnement c'est à dire pour l'essentiel la commande du déplacement des spots lumineux. Ces entrées Y1 et Y2 sont celles des étages commandant le balayage vertical des spots lumineux. Ces deux entrées étant associées directement aux moyens 5 et 6 les signaux issus de ces moyens sont représentés suivant une échelle linéaire ce qui permet une appréciation correcte des défauts ou distorsions. De préférence le tube cathodique est du type de ceux présentant une faible rémanence. Suivant une autre forme de réalisation le moyen 7 de visualisation peut être constitué par un écran vidéo.

Cette caractéristique, combinée à la caractéristique des moyens 5 et 6 qui présentent de faibles temps de réponse, fait en sorte que les modifications apportées à l'appareil en vue d'en réduire les défauts sont visualisées immédiatement. De plus, cette caractéristique est renforcée lors de l'utilisation du signal sinusoidal vobulé en fréquence. En effet, comme on le sait la fréquence de ce type de signal croît progressivement et décroît progressivement. De ce fait, la trace du spot lumineux lorsque la fréquence décroit se superposera exactement avec la trace du spot lumineux lorsque la fréquence croît.

L'image des signaux visualisés est donc particulièrement stable et les modifications apportées à l'appareil en vue de réduire les distorsions sont donc visualisées en temps réel. Comme le balayage horizontal du spot lumineux doit être progressif dans un sens comme dans l'autre lors de l'utilisation des signaux vobulés le circuit base de temps 23 du moyen de visualisation 7 fournit un signal triangulaire dont l'amplitude croît et décroît conjointement à la fréquence du signal vobulé. Il est bien évident que lors de l'utilisation de signaux non vobulés le circuit base de temps 23 du moyen 7 de visualisation fournit des signaux en dents de scie. Selon une forme préférentielle de réalisation le circuit base de temps est alimenté par le générateur 13 qui commande le générateur 12. Ce circuit base de temps 23 est commandé par un moyen 27 de visualisation des signaux suivant deux ou plusieurs périodes. Le circuit base de temps 23 est constitué par au moins une résistance 24 et par un condensateur 25 chargé par la dite résistance. Au borne de ce condensateur est prélevé le signal base de temps pour être injecté dans le moyen 7 de visualisation.

La résistance 24 reçoit du générateur 13 la tension de charge du condensateur 25 pour rattraper l'amplitude de la tension aux bornes de celui-ci en fonction de la fréquence du signal visualisé par le moyen 7. Ainsi l'écran du moyen 7 pourra être gradué linéairement, chacune des graduations correspondant à une fréquence. Le générateur de balayage 13 est constitué par deux amplificateurs 70 et 71. L'amplificateur 70 comporte un circuit de contre-réaction constitué par une résistance variable 72. Cette résistance est reliée à l'entrée non inverseuse de cet amplificateur et à sa sortie. L'entrée non inverseuse de cet amplificateur est reliée par une résistance variable 73 à la sortie de l'amplificateur 71. L'entrée inverseuse de l'amplificateur 70 est reliée à la masse. La sortie de cet amplificateur est reliée à l'entrée non inverseuse de l'amplificateur 71 par une résistance variable 71A qui règle la vitesse de balayage et par conséquent la vitesse de vobulation.

L'amplificateur 71 comporte également un circuit de contre réaction constitué par un condensateur 74. L'entrée non inverseuse de cet amplificateur est reliée à la masse par l'intermédiaire d'une résistance 75. La sortie de l'amplificateur 71 est reliée à la masse par l'intermédiaire d'une résistance ajustable 76. Sur le curseur 76A de cette résistance est prélevé le signal de commande du balayage et le signal de commande du générateur 12. Le curseur 76A de cette résistance est relié par l'intermédiaire d'une résistance 77 au générateur 12 et au circuit base de temps. Par réglage de la résistance 76 l'opérateur règle la largeur de la plage des fréquences vobulées et la largeur du balayage.

De préférence à la tension délivrée par la résistance 77 est superposée une tension continue réglable par un potentiomètre 50. Ce potentiomètre permet d'ajuster la fréquence à partir de laquelle la vobulation doit être effectuée. On comprend que grâce à un tel générateur 13 le circuit base de temps est toujours alimenté par une tension proportionnelle à la fréquence du signal visualisé.

Au générateur 13 est associé des commandes manuelles qui debrayent automatiquement la vobulation lorsqu'elles sont actionnées. Le rôle de ces commandes est de permettre à l'opérateur de ne visualiser qu'un signal de fréquence fixe.

Ces commandes sont constituées par trois interrupteurs qui permettent respectivement la visualisation d'un signal de fréquence égale à 500 hz, 1000 hz et 4000 hz et par le potentiomètre 50 qui permet de choisir une fréquence entre 20 hz et 20 000 hz.

La décharge du condensateur 25 du circuit base de temps est assurée par un interrupteur électronique 26. Cet interrupteur 26 est constitué par un transistor par exemple un «2N2222» dont la base reçoit des impulsions issues du moyen 27 précédemment cité. Sous l'effet des impulsions le transistor est rendu passant ce qui décharge le condensateur 25.

Le moyen 27 constitué par un compteur permet la visualisation de deux ou de plusieurs périodes du signal à visualiser. De préférence ce compteur visualise un nombre de périodes égal à un multiple de deux. Le nombre de périodes à visualiser est déterminé par l'injection d'une tension de commande dans une des entrées 28 de ce compteur. Le compteur 27 reçoit en dérivation le signal à visualiser. Le compteur 27 ne délivrera aucune impulsion tant que le nombre de périodes déterminées par la tension de commande n'est pas atteint.

Comme on peut le voir ce compteur comporte plusieurs entrées 28 qui déterminent chacune un nombre de périodes à visualiser. Ces entrées sont sélectionnées par un sélecteur mécanique 29 connu en soi.

La constante de temps du circuit base de temps 23 doit être modifiée en fonction du nombre de périodes à visualiser. A cet effet, ce circuit 23 comporte plusieurs résistances 24 de valeurs différentes qui sont sélectionnées en fonction du nombre de périodes à visualiser par un sélecteur 23A couplé mécaniquement au sélecteur 29.

Comme dit précédemment, le banc d'essai est équipé d'un moyen 4 pour modéliser un appareil de correction auditive propre au malentendant.

Ce moyen 4 est attaqué par un signal issu d'une source 2. Ce moyen 4 est constitué par un amplificateur 30, par un jeu de filtre 31 connu sous le nom de «égaliseur» et par au moins un transducteur électroacoustique 32 qui émet des sons destinés à être perçus par le malentendant.

Pour régler le niveau du signal injecté dans l'amplificateur 30 un potentiomètre 33 est disposé à son entrée. La sortie de cet amplificateur attaque l'égaliseur 31 qui attaque lui-même le transducteur 32.

L'égaliseur permet d'atténuer ou d'amplifier sélectivement une ou plusieurs plages de fréquences sonores. Donc avec l'aide de cet organe, l'homme de l'art sera en mesure de rétablir une audition normale chez la personne malentendante. Comme on le comprend cet organe permet de modéliser analogiquement un appareil de correction propre au malentendant.

Le transducteur électro-acoustique est constitué soit par un haut parleur et/ou par un écouteur. De préférence comme on peut le voir en fig. 1 le moyen 4 comporte un haut parleur et un écouteur.

Un de ces transducteurs est sélectionné par un sélecteur 57 comportant une position intermédiaire neutre.

Pour visualiser la courbe de réponse de l'organe 4 le signal électrique présent à la sortie de l'égaliseur est prélevé pour être injecté dans une des entrées Y1 ou Y2.

Comme le signal issu de l'égaliseur ne peut être visualisé simultanément à la visualisation des deux signaux issus des moyens 5 et 6 le banc d'essai est équipé d'un sélecteur 34 de visualisation connu en soi.

A titre d'exemple ce sélecteur est du type de ceux constitués de deux galettes montées sur un axe commun qui est actionné par un bouton de manœuvre.

Avec chaque galette coopèrent, selectivement suivant la position angulaire de celle-ci, des plots ou contacts électriques. Un des plots d'une des galettes reçoit le signal de l'égaliseur et un plot d'une des galettes et un plot de l'autre reçoivent respectivement le signal issu du moyen 5 et le signal issu du moyen 6.

Comme on le comprend une de ces galettes est reliée électriquement à l'entrée Y1 tandis que l'autre est reliée électriquement à l'entrée Y2. De préférence ce sélecteur, outre les signaux issus des moyens 5 et 6 et de l'égaliseur 31, permet également la visualisation des signaux issus des sources 2.

De préférence ces signaux sont prélevés à la sortie de l'amplificateur 30 du moyen 4. Pour dériver ce signal directement vers le moyen 7 de visualisation, un sélecteur 35 est disposé entre l'amplificateur et l'égaliseur 31.

Comme on peut le voir en figure 1, le band d'essai peut recevoir deux appareils de correction auditive.

A cet effet, le banc d'essai est doté de deux potentiomètres 15, de deux moyens 3, de deux moyens 4, de deux moyens 5, et de deux moyens 6. Le sélecteur 34 permet de sélectionner simultanément outre les signaux électriques issus des moyens 5 et 6 associés à un appareil 1, les signaux électriques issus de la source 2, les signaux électriques issus des deux moyens 5, les signaux électriques issus des deux moyens 6 et les signaux électriques issus des moyens 4.

Pour la mise au point de l'appareil de correction l'homme de l'art a besoin de connaître le gain moyen de cet appareil lorsqu'il est soumis au signal vobulé et le gain de celui-ci pour un signal sinusoidal de fréquence donnée. Comme on le sait, le gain d'un étage électro-acoustique est la différence en décibels entre le niveau du signal à l'entrée de cet étage et le niveau du signal à la sortie de cet étage. Le gain de l'appareil de correction sera donc égal à la différence entre le niveau du signal issu du moyen 5 et le niveau du signal issu du moyen 6.

Pour la mesure de ce gain le banc d'essai est doté de deux vu-mètres 56 dont un est couplé avec une galette du sélecteur 34 et dont l'autre est couplé avec l'autre galette de ce sélecteur. Comme les signaux issus des moyens 5 et 6 associés à un même appareil sont associés chacun à une galette un de ces vu-mètres indiquera le niveau du signal du moyen 5 et l'autre le niveau du signal du moyen 6.

Selon la forme de réalisation les vu-mètres 56 présentent une dynamique de vingt décibels. Il est donc nécessaire d'atténuer le signal à l'entrée de ces vu-mètres et de prévoir pour chacun trois plages de mesures par exemple de 60 décibels à 80 décibels puis 80 décibels à 100 décibels et 100 décibels à 120 décibels. Pour chaque vu-mètre ces plages de mesure sont sélectionnées par un sélecteur multi-positions 36. De préférence à chacune de ces plages de mesure est associée une diode électroluminescente 37 pour indiquer à l'homme de l'art qu'elle est la plage de mesure qu'il vient de sélectionner. Il est à noter qu'à la sortie de ces vu-mètres le signal injecté dans ceux-ci, ce signal étant soit le signal issu du moyen 5 ou soit le signal issu du moyen 6, est délivré suivant une échelle logarithmique. Il est parfois utile à l'homme de l'art de visualiser ce signal suivant une forme logarithmique et non plus linéaire.

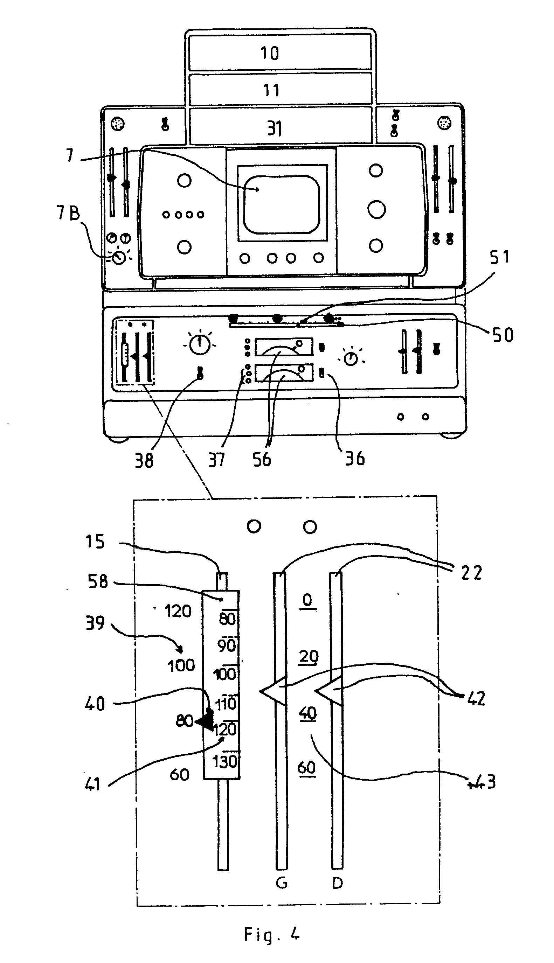

Pour sélectionner ce mode de représentation logarithmique ou linéaire un sélecteur 38 est associé à l'entrée Y1 ou Y2 correspondante du moyen 7. Ce sélecteur à deux positions sélectionne soit la sortie logarithmique du vu-mètre correspondant, ou soit la galette correspondante du sélecteur 34. En fig. 4 n'apparaît qu'un seul potentiomètre 15 bien que le banc d'essai selon la forme de réalisation présenté reçoit deux appareils 1.

En se référant à la fig. 1 on peut voir que ce potentiomètre 15 est en fait un double potentiomètre comportant donc deux pistes ajustant le niveau du signal à l'entrée des moyens 3 et 4. Il est bien évident que ce potentiomètre est conçu de manière à délivrer deux signaux de niveau égal. Les deux potentiomètres 22 visibles en fig. 4 sont associés chacun à un appareil de correction 1. Comme il est nécessaire d'atténuer le signal issu du moyen 6 à l'aide du potentiomètre 22 il est nécessaire pour la mesure du gain de l'appareil de tenir compte de cette atténuation.

Pour faciliter cette mesure les potentiomètres 15 et 22 sont disposés les uns à côté des autres, comportent chacun un index et coulissent devant une échelle graduée. De plus la tige de manœuvre du potentiomètre 15 comporte une réglette graduée 58 en regard de laquelle viennent se disposer les index des potentiomètres 22. Les échelles graduées des potentiomètres 15 et 22 et l'échelle graduée de la réglette 58 se développent suivant une même direction et sont établis de manière à indiquer quelle que soit la position des potentiomètres, le niveau du signal perçu par l'appareil, le niveau du signal émis par l'appareil et le niveau d'atténuation donc le gain de l'appareil. Comme on peut le voir en fig. 4, l'échelle graduée 39 du potentiomètre 15 indique des valeurs corissantes du bas vers le haut. Ces valeurs sont relatives au niveau du signal perçu par l'appareil. Le réglage du niveau du signal est opéré en disposant un index 40 face à une graduation de l'échelle 39. Cet index 40 est porté par la réglette 58. L'échelle graduée 41 de la réglette 58 est relative au niveau du signal émis par l'appareil 1. Les valeurs marquées sur cette réglette sont croissantes du haut vers le bas. Face à une des graduations de la réglette graduée 58 viennent se disposer deux index 42 portés respectivement par chaque potentiomètre 22. De plus ces index gradués viennent se disposer chacun en regard d'une échelle graduée relative au niveau d'atténuation du signal émis par le moyen 6 et donc au gain de l'appareil. Les valeurs marquées sur cette échelle sont croissantes du haut vers le bas.

En prenant maintenant un exemple on va décrire l'utilisation des différentes échelles graduées ainsi que des vu-mètres. Par exemple l'homme de l'art soumet l'appareil à un signal sonore de niveau égal à 80 décibels. Il sélectione tout d'abord la plage de mesure adaptée à l'aide du sélecteur 36. Par exemple il sélectionne la plage de mesure comprise entre 60 et 80 décibels. Par la suite en agissant sur le potentiomètre 15 il amènera l'index 40 en regard de la graduation marquée «80» de l'échelle graduée 39.

L'aiguille du vu-mètre mesurant le niveau du signal perçu par l'appareil déviera et se disposera en regard de la graduation marquée «0» du dit vu-mètre. A l'aide d'un des potentiomètres 22 qui règle le niveau d'atténuation du signal émis par l'appareil, l'homme de l'art dévie l'aiguille du second vu-mètre affecté à la mesure de ce signal en regard de la graduation «0». La lecture du gain de l'appareil sera indiquée par l'index 42 et par l'échelle graduée 43. De plus cet index qui est disposé en regard de l'échelle graduée 41 de la réglette 58 indique le niveau du signal de sortie. Il est bien évident que préalablement l'homme de l'art a ajusté la plage de mesure du second vu-mètre. Lorsque l'homme de l'art emploie des signaux vobulés en fréquence il ne peut mesurer qu'un gain moyen. Cependant il est intéressant de connaître le gain de l'appareil à une fréquence donnée. Dans ce but, l'homme de l'art agit sur le moyen 22 pour appliquer au générateur de fonctions une tension continue dont l'amplitude, qui règle la fréquence du signal, est commandée par le potentiomètre 50.

Le potentiomètre 50 comme on peut le voir en fig. 4 est disposé sous le tube cathodique et se manœuvre horizontalement. Ce potentiomètre comporte un index qui coulisse face à une échelle graduée 51 relative à la fréquence du signal sinusoidal.

Comme dit précédemment le banc d'essai est équipé d'un moyen pour mémoriser notamment la réponse du modèle d'appareil de correction. Par exemple ce moyen pourra être constitué par un appareil photographique. Par comparaison entre la courbe mémorisée du modèle d'appareil et la réponse de l'appareil l'homme de l'art sera en mesure de procéder à tous réglages utiles visant à adapter le dit appareil à l'audition du patient. De plus grâce au fait de mémoriser la réponse du modèle d'appareil l'homme de l'art sera en mesure de suivre l'évolution de la surdité du malentendant et pourra également régler l'appareil de correction, sans la présence du malentendant. Avantageusement le banc d'essai peut être doté d'une imprimante inscrivant les données utiles à l'opérateur.

Le banc d'essai selon l'invention peut avantageusement comporter une numerisation de valeurs obtenue par exemple par un convertisseur analogique numérique permettant l'affichage des valeurs de la fréquence, des décibels et du pourcentage de distorsion. Le banc d'essai est également équipé de moyen 52 à l'usage de l'homme de l'art permettant l'écoute de différents signaux. Ces moyens sont constitués par un sélecteur d'écoute 53 qui sélectionne un des signaux émis par les différents moyens du banc d'essai par des étages d'amplification 54 et par des transducteurs électro-acoustiques 55 à l'usage de l'opérateur ou du patient.

Le banc d'essai selon la présente invention est plus particulièrement destiné à la mise au point des appareils de correction auditive mais il va de soi qu'il pourra trouver son emploi dans la mise au point de toute chaîne électro-acoustique.

## Revendications

1. Banc d'essai pour la mise au point des chaînes électro-acoustiques et notamment des appareils (1) de correction auditive en sorte que ceux-ci restituent avec fidélité le timbre et la hauteur des sons perçus, et restituent ces sons avec plus ou moins d'intensité en fonction de l'audition du malentendant, le dit appareil comportant:

   - plusieurs sources (2) de production d'un signal électrique,
   - au moins un moyen (3) de transformation du signal électrique en signal sonore, le dit signal sonore étant reçu par l'appareil (1),
   - au moins un moyen (6) pour transformer en signal électrique le signal sonore émis par l'appareil,
   - et un moyen (7) de visualisation des signaux électriques, le dit banc d'essai étant caractérisé par:
   - au moins un ensemble de moyens (4) pour modéliser un appareil de correction auditive propre au malentendant,
   - par au moins un moyen (5) pour transformer en signal électrique le signal sonore reçu par l'appareil,
   - par l'utilisation du moyen (7) de visualisation pour visualiser les signaux électriques délivrés par l'ensemble de moyens (4) pour modéliser l'appareil de correction auditive propre au malentendant, par le moyen (5) pour transformer en signal électrique le signal sonore recu par l'appareil (1), et par le moyen (6) pour transformer en signal électrique le signal sonore émis par l'appareil (1),
   – et par des moyens de mémorisation des signaux électriques visualisés.

2. Banc d'essai selon la revendication 1 caractérisé en ce que les signaux sont visualisés suivant une échelle linéaire.

3. Banc d'essai selon la revendication 1 caractérisé en ce que les sources (2) sont constituées par au moins un moyen générant un signal audio-fréquence et par au moins un moyen (12) générant un signal carré, un signal triangulaire, un train d'impulsions et un signal sinusoidal de fréquence fixe ou variable.

4. Banc d'essai selon les revendications 1 et 2 comportant un moyen (12) qui génére un signal et une unité de visualisation (7) comportant un circuit (23) de base de temps caractérisé par un générateur de tension (13) qui commande la dite base de temps et le générateur (12).

5. Banc d'essai selon les revendications 1 et 4 caractérisé en ce que le signal est vobulé en fréquence, c'est-à-dire que la fréquence de ce signal croît et décroît de manière cyclique et que ce signal est d'amplitude constante.

6. Banc d'essai selon les revendications 4 et 5 caractérisé en ce que le générateur de tension (13) produit un signal triangulaire pour commander la vobulation du signal.

7. Banc d'essai selon la revendication 1 comportant un moyen de visualisation (7) doté d'une tube cathodique et comportant le moyen (6) pour transformer le signal sonore émis par l'appareil en signal électrique caractérisé en ce que le tube cathodique est du type de ceux présentant une faible rémanence et que le moyen (6) pour transformer en signal électrique le signal sonore émis par l'appareil est du type de ceux présentant de faible temps de réponse en sorte que les effets des corrections apportées à l'appareil pour en réduire les défauts ou distorsions soient visualisés immediatement.

8. Banc d'essai selon la revendication 1 caractérisé en ce qu'il est doté d'un coupleur acoustique (60) couplant acoustiquement un haut parleur (17) du moyen (3) de transformation du signal électrique en signal sonore reçu par l'appareil, avec un microphone (18) du moyen (5) de transformation un signal électrique le signal sonore reçu par l'appareil et avec le microphone de l'appareil et qu'il est doté d'un second coupleur acoustique (61) couplant acoustiquement le haut parleur du dit appareil (1) et le microphone (19) du moyen (6) pour transformer en signal électrique le signal sonore émis par l'appareil.

9. Banc d'essai selon la revendication 8 caractérisé en ce que le coupleur (60) est constitué par deux enceintes tubulaires (62; 63) recevent le haut parleur (17) et le microphone (18) comportant chacune un fond (64) et une embouchure filetée pour se visser l'une avec l'autre.

10. Banc d'essai selon les revendications 8 et 9 caractérisé en ce que les enceintes (62; 63) du coupleur (60) reçoivent de la mousse.

11. Banc d'essai selon les revendications 8 et 9 caractérisé par un tube reliant acoustiquement une des l'enceintes (63) au microphone de l'appareil (1).

12. Banc d'essai selon les revendications 8 et 9 caractérisé en ce que chaque enceinte (62; 63) du coupleur est dotée d'une série d'orifices transversaux (67) angulairement espacés, ménagés dans sa paroi cylindrique et d'une bague (68) emmanchée en frottement doux sur la dite paroi pour venir obturer plus ou moins les dits orifices.

13. Banc d'essai selon la revendication 1 caractérisé par un potentiomètre (15) pour régler le volume des signaux des sources (2) dans le moyen (3) de transformation du signal électrique en signal sonore reçu par l'appareil (1) et dans le moyen (4) de modélisation de l'appareil de correction auditive.

14. Banc d'essai selon la revendication 1 comportant un moyen (6) pour transformer le signal sonore émis par l'appareil en signal électrique, le dit moyen (6) pour transformer en signal électrique le signal sonore émis par l'appareil étant constitué par un microphone (19) et par un amplificateur (21) caractérisé en ce qu'un potentiomètre (22) est disposé entre le microphone et l'amplificateur.

15. Banc d'essai selon les revendications 13 et 14 caractérisé en ce que les potentiomètres (15) réglant le volume des sources (2) de production de signal électrique et le potentiomètre (22) de chaque moyen (6) pour transformer en signal électrique le signal sonore émis par l'appareil, sont disposés les uns à côté des autres, que ces potentiomètres comportent chacun un index, que ces potentiomètres coulissent chacun devant une échelle graduée et que les potentiomètres (15) comportent chacun une réglette graduée (58) en regard de laquelle viennent se disposer les index (42) des potentiomètres (22).

16. Banc d'essai selon la revendication 15 caractérisé en ce que les échelles graduées des potentiomètres (15) réglant le volume des sources (2) et du potentiomètre (22) de chaque moyen (6) pour transformer en signal électrique le signal sonore émis par l'appareil, se développent suivant une même direction et qu'elles sont établies de manière à indiquer le niveau du signal perçu par l'appareil, le niveau du signal émis par l'appareil et le gain de l'appareil quelle que soit la position des dit potentiomètres.

17. Banc d'essai selon la revendication 1 caractérisé par un sélecteur de visualisation (34).

18. Banc d'essai selon la revendication 1 dont l'unité de visualisation comporte un circuit base de temps caractérisé en ce que le dit curcuit base de temps est alimenté en tension par le générateur de tension (13).

**Patentansprüche**

1. Prüfstand für die Einstellung von elektroakustischen Anlagen und insbesondere Gehörhilfsgeräten (1), sodass diese die Klangfarbe und die Höhe der wahrgenommenen Töne treu wiedergeben, mit mehr oder weniger Lautstärke je nach dem Hörvermögen des Hörbehinderten, wobei das besagte Gerät enthält:

- mehrere Quellen (2) für die Erzeugung eines elektrischen Signals,
- mindestens ein Mittel (3) der Umwandlung des elektrischen Signals in ein akustisches Signal, wobei das besagte akustische Signal vom Gerät (1) aufgenommen wird,
- mindestens ein Mittel (6) für die Umwandlung des vom Gerät ausgesandten akustischen Signals in ein elektrisches Signal,
- und ein Mittel (7) für die Anzeige elektrischer Signale, wobei besagter Prüfstand gekennzeichnet ist
- durch mindestens eine Einheit von Mitteln (4) für die Modellisierung eines dem Hörbehinderten eigenen Hörhilfsgeräts,
- durch mindestens ein Mittel (5), um das vom Gerät empfangene akustische Signal in ein elektrisches Signal umzuwandeln,
- durch die Verwendung des Anzeigemittels (7) für die Sichtbarmachung der elektrischen Signale, die von der Gesamtheit der Mittel (4) für die Modellisierung der vom Hörhilfsgerät für Hörbehinderte abgegeben werden,
- durch mindestens ein Mittel (5) für die Umwandlung des vom Gerät (1) empfangenen akustischen Signals in ein elektrisches Signal und durch das Mittel (6) für die Umwandlung des vom Gerät (1) ausgegebenen akustischen Signals in ein elektrisches Signal,
- und durch Mittel für die Speicherung der sichtbar gemachten elektrischen Signale.

2. Prüfstand gemäss Anspruch 1, dadurch gekennzeichnet, dass die Signale in einer linearen Skala angezeigt werden.

3. Prüfstand gemäss Anspruch 1, dadurch gekennzeichnet, dass die Quellen (2) aus mindestens einem Mittel bestehen, das ein Hörfrequenzsignal erzeugt, und mindestens einem Mittel (12), das ein quadratisches Signal, ein Dreieckssignal, eine Impulsfolge und ein sinusförmiges Signal fester oder veränderlicher Frequenz erzeugt.

4. Prüfstand gemäss den Anspruch 1 und 2 mit einem Mittel (12) das ein Signal erzeugt, und einer Anzeigeeinheit (7), die eine Zeitbasisschaltung (23) enthält, gekennzeichnet durch einen Spannungsgenerator (13), der die besagte Zeitbasis und den Generator (12) steuert.

5. Prüfstand gemäss Anspruch 1 und 4, dadurch gekennzeichnet, dass das Signal frequenzgewobbelt wird, das heisst, dass die Frequenz des Signals zyklisch zu- und abnimmt und dass die Amplitude des Signals konstant ist.

6. Prüfstand gemäss Anspruch 4 und 5, dadurch gekennzeichnet, dass der Spannungsgenerator (13) ein Dreiecksignal für die Steuerung der Signalwobbelung erzeugt.

7. Prüfstand gemäss Anspruch 1, mit einem Anzeigemittel (7), das über eine Kathodenstrahlröhre verfügt und mit dem Mittel (6) für die Umwandlung des vom Gerät ausgegebenen akustischen Signals in ein elektrisches Signal, dadurch gekennzeichnet, dass die Kathodenstrahlröhre vom Typ mit schwacher Remanenz ist, und dass das Mittel (6) für die Umwandlung des vom Gerät ausgegebenen akustischen Signals in ein elektrisches Signal vom Typ mit kurzer Ansprechzeit ist, sodass die Auswirkungen der Korrekturen, die am Gerät vorgenommen werden, um seine Fehler oder Verzerrungen zu reduzieren, sofort angezeigt werden.

8. Prüfstand gemäss Anspruch 1, dadurch gekennzeichnet, dass er mit einem akustischen Koppler (60) versehen ist, der akustisch einen Lautsprecher (17) des Mittels (3) für die Umwandlung des elektrischen Signals in das vom Gerät aufgenommene akustische Signal verbindet mit einem Mikrofon (18) des Mittels (5) für die Umwandlung eines vom Gerät mit dem Mikrofon des Gerätes empfangenen akustischen Signal in ein elektrisches Signal und mit dem Mikrofon des Gerätes, und dass er mit einem zweiten akustischen Koppler (61) versehen ist, der akustisch den Lautsprecher des besagten Gerätes (1) mit dem Mikrofon (19) des Mittels (6) für die Umwandlung des vom Gerät ausgesandten akustischen Signals in ein elektrisches Signal verbindet.

9. Prüfstand gemäss Anspruch 8, dadurch gekennzeichnet, dass der Koppler (60) aus zwei rohrförmigen Kammern (62 und 63) besteht, die den Lautsprecher (17) und das Mikrofon (18) aufnehmen und je einen Boden (64) und ein Gewindeendstück enthalten, um sich aneinander anschrauben zu lassen.

10. Prüfstand gemäss den Ansprüchen 8 und 9, dadurch gekennzeichnet, dass die beiden Lautsprecher (62 und 63) des Kopplers (60) mit Schaum versehen werden.

11. Prüfstand gemäss Anspruch 8 und 9, gekennzeichnet durch eine Röhre, die akustisch eine der beiden Lautsprecher (62, 63) mit dem Mikrofon des Gerätes (1) verbindet.

12. Prüfstand gemäss Anspruch 8 und 9, dadurch gekennzeichnet, dass jede Kammer des Kopplers mit einer Reihe von Queröffnungen (67) mit Winkelabständen versehen ist, Öffnungen, die in die zylindrische Wand eingebracht wurden, und mit einem Ring (68), der vorsichtig auf die besagte Wand aufgebracht wird, um die besagten Öffnungen mehr oder weniger dicht zu schliessen.

13. Prüfstand gemäss Anspruch 1, durch ein Potentiometer (15) gekennzeichnet, um die Särke der Signale der Quelle (2) im Mittel (3) für die Umwandlung des elektrischen Signals in ein vom Gerät (1) und im Mittel (4) für die Modellierung des Gehörhilfsgerätes aufgenommene akustisches Signal einzustellen.

14. Prüfstand gemäss Anspruch 1 mit einem Mittel (6) für die Umwandlung des vom Gerät ausgegebenen akustischen Signals in ein elektrisches Signal, wobei besagtes Mittel (6) für die Umwandlung des vom Gerät ausgegebenen akustischen Signals in ein elektrisches Signal aus einem Mikrofon (19) und einem Verstärker (21) besteht, dadurch gekennzeichnet, dass zwischen dem Mikrofon und dem Verstärker (21) ein Potentiometer (22) angeordnet ist.

15. Prüfstand gemäss Anspruch 13 und 14, dadurch gekennzeichnet, dass die Potentiometer (15), mit denen die Stärke der Quellen (2) für die Erzeugung des elektrischen Signals eingestellt wird, und das Potentiometer (22) eines jeden Mittels (6) für die Umwandlung des vom Gerät ausgegebenen akustischen Signals in ein elektrisches Signal nebeneinander angeordnet sind, dass diese Potentiometer jeweils eine Kennmarke enthalten, dass sich diese Potentiometer vor einer Skala mit Stricheinteilung verschieben lassen und dass die Potentiometer (15) jeweils eine Skaleneinteilung (58) enthalten, vor der die Kennmarken (42) der Potentiometer (22) angeordnet werden.

16. Prüfstand gemäss Anspruch 15, dadurch gekennzeichnet, dass sich die Messskalen der Potentiometer (15), mit denen die Lautstärke der Quellen (2) und des Potentiometers (22) eines jeden Mittels für die Umwandlung des akustischen Signals in ein elektrisches Signal eingestellt werden, in der gleichen Richtung erstrecken und dass sie so erstellt sind, dass sie die Stärke des vom Gerät aufgenommenen Signals, die Stärke des vom Gerät ausgegebenen Signals und die Verstärkung durch das Gerät unabhängig von der Position der besagten Potentiometer anzeigen.

17. Prüfstand gemäss Anspruch 1, durch einen Anzeigewählschalter (34) gekennzeichnet.

18. Prüfstand gemäss Anspruch 1, dessen Anzeigeeinheit eine Zeitbasisschaltung enthält, dadurch gekennzeichnet, dass die besagte Zeitbasisschaltung durch den Spannungsgenerator (13) mit Spannung gespeist wird.

## Claims

1. A test bench for tuning electroacoustic chains and more particularly hearing correction appliances (1) so that they faithfully restore the timbre and the pitch of the sounds received, and restore these sounds with greater or lesser intensity depending on the hearing of the hard of hearing person, said appliance comprising:
   - several electric signal production sources (2),
   - at least one means (3) for transforming the electric signal into a sound signal, said sound signal being received by the appliance (1),
   - at least one means (6) for transforming the sound signal emitted by the appliance into an electric signal,
   - and a means (7) for displaying the electric signals, the test bench being characterized by:
   - at least one assembly of means (4) for forming a model of a hearing correction appliance proper to the hard of hearing person,
   - by at least one means (5) for transforming the sound signal received by the appliance into an electrical signal,
   - by the use of the display means (7) for displaying the electric signals delivered by the assembly of means (4) for forming the model of a hearing correction appliance proper to the hard of hearing person,
   - by the means (5) for transforming the sound signal received by the apparatus (1) into an electric signal and by the means (6) for transforming the sound signal emitted by the appliance (1) into an electric signal,
   - and by means for storing the electric signals displayed.

2. The test bench according to claim 1, characterized in that ghe signals are displayed in accordance with a linear scale.

3. The test bench according to claim 1, characterized in that the sources (2) are formed by at least one means generating an audio frequenzy signal and by at least one means (12) generating a square signal, a triangular signal, a pulse train and a sinusoidal signal of fixed or variable frequency.

4. The test bench according to claims 1 and 2 comprising a means (12) which generates a signal and a display unit (7) including a time base circuit (23), characterized by a voltage generator (13) which controls said time base and the generator (12).

5. The test bench according to claims 1 and 4, characterized in that the signal is frequency wobbulated, that is to say that the frequency of this signal increases and decreases cyclically and that this signal is of constant amplitude.

6. The test bench according to claims 4 and 5, characterized in that the voltage generator (13) produces a triangular signal for controlling the wobbulation of the signal.

7. The test bench according to claim 1 including a display means (7) having a cathode ray tube and including the means (6) for transforming the sound signal emitted by the appliance into an electric signal, characterized in that the cathode ray tube is of the type having low remanence and that the means (6) for transforming the sound signal emitted by the appliance into an electric signal is of the type having a low resonse time so that the effects of the corrections made to the appliance so as to reduce the defects or distortions are immediately displayed.

8. The test bench according to claim 1, characterized in that it is provided with an acoustic coupler (60) acoustically coupling a loud speaker (17) of the means (3) for transforming the electric signal into a sound signal received by the appliance, with a microphone (18) of the means (5) for transforming into an electric signal the sound signal received by the appliance and with the microphone of the appliance and in that it is provided with a second acoustic coupler (61) acoustically coupling the loud speaker of said appliance (1) and the microphone (19) of the means (6) for transforming the sound signal emitted by the appliance into an electric signal.

9. The test bench according to claim 8, characterized in that the coupler (60) is formed by two tubular enclosures (62, 63) receiving the loud speaker (17) and the microphone (18) each having a bottom (64) and a threaded mouth piece for screwing together.

10. The test bench according to claims 8 and 9, characterized in that the enclosures (62, 63) of the coupler, (60) receive foam.

11. The test bench according to claims 8 and 9, characterized by a tube acoustically connecting one of the enclosures (63) to the microphone of the appliance (1).

12. The text bench according to claims 8 and 9, characterized in that each enclosure (62, 63) of the coupler is provided with a series of angularly spaced transverse orifices (67) formed in its cylindrical wall and a ring (68) fitted on said wall with an easy fit so as to close said orifices to a greater or lesser extent.

13. The test bench according to claim 1, characterized by a potentiometer (15) for adjusting the volume of the signals of the sources (2) in the means (3) for transforming the electric signal into a sound signal received by the appliance (1) and in the means (4) for forming a model of the hearing correction appliance.

14. The test bench according to claim 1, including a means (6) for transforming the sound signal emitted by the appliance into an electric signal, said means (6) for transforming the sound signal emitted by the appliance into an electric signal being formed by a microphone (19) and an amplifier (21), characterized in that a potentiometer (22) is disposed between the microphone and the amplifier.

15. The test bench according to claims 13 and 14, characterized in that the potentiometers (15) adjusting the volume of the electric signal production sources (2) and the potentiometer (22) of each means (6) for transforming the sound signal emitted by the appliance into an electric signal are disposed at the side of each other, in that these potentiometers each have a pointer, in that these potentiometers each slide in front of a graduated scale and in that the potentiometers (15) each have a graduated strip (58) in front of which are disposed the pointers (42) of the potentiometers (22).

16. The test bench according to claim 15, characterized in that the graduated scales of the potentiometers (15) and of the potentiometers (22) of each means (6) for transforming the sound signal emitted by the appliance into an electric signal extend in the same direction and in that they are formed so as to indicate the level of the signal received by the appliance, the level of the signal emitted by the appliance and the gain of the appliance whatever the position of said potentiometers.

17. The test bench according to claim 1, characterized by a display selector (34).

18. The test bench according to claim 1, whose display unit includes a time base circuit, characterized in that said time base circuit is fed with voltage by the voltage generator (13).

Fig. 1

Fig. 2

Fig. 3

Fig. 4

fig.5

fig.6     Coupe AA

fig.7